# FASCICULE DE BREVET EUROPEEN

(11) **EP 3 005 989 B1**
(45) Date de publication et mention de la délivrance du brevet: **19.04.2017**
(21) Numéro de dépôt: 14188340.5
(22) Date de dépôt: 09.10.2014
(51) Int. Cl.: A61F 2/42

(54) **Implant de resurfaçage du radius distal**
Implantat für das Resurfacing des distalen Radius
Implant for resurfacing the distal radius

(43) Date de publication de la demande: 13.04.2016
(73) Titulaire: Groupe Lepine, 69730 Genay (FR); Université Claude Bernard Lyon 1, 69922 Villeurbanne (FR); Hospices Civils De Lyon, 69223 Lyon Cedex 02 (FR)
(72) Inventeur: Herzberg, Guillaume, 69006 Lyon (FR); Morel, David, 43190 Tence (FR)
(74) Mandataire: Jeannet, Olivier

(56) Documents cités:
- EP-A2- 2 559 407
- DE-U1- 29 500 476
- DE-U1-202004 020 558
- GB-A- 2 392 097

## Description

La présente invention concerne un implant de resurfaçage du radius distal. Elle concerne également une prothèse à articulation radio-ulnaire prothétique, incluant cet implant.

Pour réaliser la reconstruction d'un radius distal, ou pour remplacer l'articulation radio-carpienne native en cas de fracture complexe, en particulier chez un patient âgé pour qui l'ostéosynthèse est vouée à l'échec, il est connu d'utiliser un implant de resurfaçage du radius distal, monobloc et à un seul composant. Cet implant comprend un plateau formant une surface d'articulation concave, destinée à venir directement en relation d'articulation avec les os de la première rangée d'os du carpe, et une tige d'ancrage effilée solidaire de ce plateau, destinée à être insérée dans le canal médullaire de la portion distale du radius.

Dans cet implant connu, la surface d'articulation est bordée sur sa périphérie par un rebord très enveloppant, recourbé vers l'intérieur de ladite surface d'articulation, et le plateau présente, vu de face, une forme en goutte d'eau : ce plateau est en effet arrondi sur sa zone destinée à se trouver, après implantation, sur le côté interne d'un radius, et est pointu sur sa zone destinée à se trouver, après implantation, sur le côté externe. La tige d'ancrage, quant à elle, est aplatie et présente un bord concave sur son côté destiné à se trouver sur le côté interne d'un radius après implantation, et un bord rectiligne sur son côté destiné à se trouver sur le côté externe.

Cet implant ne donne satisfaction que si les rapports entre le radius et l'ulna peuvent rester cohérents, soit par les surfaces radio-ulnaires distales natives conservées, soit par une résection de la tête ulnaire.

La présente invention a pour objectif principal de remédier à cet inconvénient.

Le document GB 2 392 097 A décrit un implant de resurfaçage du radius distal, monobloc et à un seul composant, comprenant un plateau formant une surface d'articulation concave et une tige d'ancrage effilée, solidaire de ce plateau, destinée à être insérée dans le canal médullaire de l'extrémité distale du radius ; le plateau présente vu de face, une forme ovale occupée essentiellement par la surface d'articulation concave, et est dépourvu de rebord périphérique enveloppant ; la tige d'ancrage présente une double inclinaison par rapport au plateau, à savoir une inclinaison vers le côté interne de l'implant et une inclinaison vers le côté palmaire de l'implant.

L'implant décrit par ce document ne permet pas de remédier à l'inconvénient précité.

Il existe par ailleurs des prothèses pour articulation radio-ulnaire, soit de resurfaçage, soit semi-contraintes, ne donnant pas non plus parfaitement satisfaction.

L'implant concerné est, de manière connue en soi, monobloc et à un seul composant, et comprend un plateau formant une surface d'articulation concave et une tige d'ancrage effilée, solidaire de ce plateau, destinée à être insérée dans le canal médullaire de l'extrémité distale du radius ; le plateau présente vu de face, une forme ovale occupée essentiellement par la surface d'articulation concave, et est dépourvu de rebord périphérique enveloppant ; la tige d'ancrage présente une double inclinaison par rapport au plateau, à savoir une inclinaison vers le côté interne de l'implant et une inclinaison vers le côté palmaire de l'implant.

### Selon l'invention,

- ladite surface d'articulation concave est destinée à venir directement en relation d'articulation avec les os de la première rangée d'os du carpe,
- le plateau est symétrique par rapport à un axe de symétrie ;
- l'inclinaison de la tige d'ancrage vers le côté interne est telle que l'axe central de cette tige forme un angle allant de 6 à 16° avec cet axe de symétrie, et l'inclinaison de la tige d'ancrage vers le côté palmaire de l'implant est telle que l'axe central de cette tige forme un angle allant de 4 à 12° avec cet axe de symétrie.

Il sera compris que les expressions "côté interne" et "côté palmaire" font respectivement référence aux côtés de l'implant se trouvant respectivement sur le côté interne et sur le côté palmaire d'un radius après mise en place de l'implant sur ce radius.

Sur l'implant selon l'invention, la combinaison de la forme ovale du plateau et de la double inclinaison de la tige d'ancrage par rapport à l'axe de symétrie de ce plateau s'avère conférer à cet implant une adaptation à l'anatomie de l'articulation radio-carpienne et permettre de restaurer cette articulation. De plus, cette forme spécifique favorise la stabilité de la première rangée d'os du carpe par rapport à l'implant et assure une mobilité optimale de ces os par rapport audit plateau.

Cette configuration permet une résection a minima du radius distal et permet soit de préserver l'échancrure sigmoïde osseuse de ce radius distal et l'articulation du radius distal à la tête de l'ulna distal (articulation radio-ulnaire distale), soit d'associer à la mise en place de l'implant une résection arthroplastique de la tête ulnaire, dite intervention de "Darrach".

De préférence, l'inclinaison de la tige vers le côté interne de l'implant est telle que l'axe central de cette tige forme un angle de 11° avec cet axe de symétrie, et l'inclinaison de la tige vers le côté palmaire de l'implant est telle que l'axe central de cette tige forme un angle de 8° avec cet axe de symétrie.

La longueur du plateau peut être comprise entre 25 mm et 35 mm et sa largeur peut être comprise entre 13 mm et 23 mm.

Avantageusement, le plateau présente, tant dans le sens de sa longueur que dans le sens de sa largeur, une face de forme arrondie et convexe sur son côté relié à la tige d'ancrage.

Cette forme permet à l'implant de venir s'insérer au mieux sur l'extrémité distale d'un radius.

La tige d'ancrage présente avantageusement une portion métaphysaire de forme conique aplatie et une portion diaphysaire de forme générale cylindrique.

Cette forme assure un bon remplissage de la portion métaphysaire du radius, et donc une bonne stabilité de l'implant dans le canal médullaire de ce radius.

La portion métaphysaire de la tige peut présenter des faces planes dont une est tournée vers le côté dorsal de l'implant et l'autre est tournée vers le côté palmaire de l'implant. Ces faces planes renforcent la stabilité de la tige par rapport à l'os.

La tige peut avoir une longueur comprise entre 30 mm et 50 mm. Cette longueur relativement limitée confère à l'implant un caractère peu invasif.

La tige d'ancrage peut en outre comporter un revêtement de surface favorisant l'ostéo-intégration, en particulier un revêtement bicouche de titane poreux et d'Hydroxyapatite.

De préférence, l'implant comprend également deux ailettes d'ancrage au niveau de la zone de raccordement du plateau et de la tige d'ancrage, une ailette étant située sur le côté interne de l'implant et l'autre ailette étant située sur le côté externe de cet implant.

Ces deux ailettes, situées sur les deux côtés opposés interne et externe de l'implant, assurent un calage de l'implant par rapport à l'os spongieux métaphysaire du radius.

Pour le calage en rotation, chaque ailette présente avantageusement une forme à deux bords plus ou moins perpendiculaires, raccordés l'un à l'autre par un bord intermédiaire arrondi.

Avantageusement, au moins une de ces ailettes présente un bord périphérique acéré, rendant possible l'insertion de l'implant dans un radius par impaction.

De préférence, au moins une ailette, en particulier celle située sur le côté interne de l'implant, est percée d'un trou de passage d'un ou plusieurs fils de suture.

Ce ou ces fils de suture permettent de remettre en place et de maintenir des fragments de l'extrémité distale du radius, le cas échéant, selon l'étendue de la fracture à traiter.

L'implant selon l'invention peut être dépourvu d'articulation radio-ulnaire, ou peut inclure une prothèse à articulation radio-ulnaire prothétique. Dans ce deuxième cas, avantageusement :
- l'implant de resurfaçage du radius distal est solidaire d'une portion annulaire définissant intérieurement un siège en forme de portion de sphère, et
- la prothèse comprend :
   une tige ulnaire incluant une portion distale médullaire, destinée à être insérée dans le canal médullaire de l'ulna, et un pion proximal de forme générale cylindrique ; et
   une tête d'articulation de la tige ulnaire par rapport à l'implant de resurfaçage du radius distal, présentant une face extérieure sphérique ou en portion de sphère, destinée à être reçue contre ledit siège avec glissement, et un alésage axial, permettant l'engagement de cette tête sur ledit pion, de manière ajustée mais avec possibilité de coulissement.

Le glissement de ladite tête contre ledit siège permet un débattement de la tige ulnaire par rapport à l'implant de resurfaçage du radius distal et, simultanément, le coulissement de cette tête le long dudit pion permet un coulissement de cette tige ulnaire par rapport à l'implant de resurfaçage du radius distal selon l'axe de ce pion.

La combinaison de ces deux possibilités de mouvements permet l'obtention d'une prothèse à articulation radio-ulnaire semi-contrainte donnant satisfaction en termes de restitution du mouvement naturel et de facilité d'implantation.

De préférence, le débattement de la tige ulnaire par rapport à l'implant de resurfaçage du radius distal est de l'ordre de 20 degrés par rapport à une position neutre d'alignement de l'axe de la tige ulnaire avec l'axe de révolution dudit siège, soit un débattement d'environ 40 degrés d'une position d'inclinaison maximale de la tige ulnaire à une position d'inclinaison maximale opposée.

De préférence, ladite portion annulaire prolonge l'une des deux ailettes d'ancrage précitée et forme corps avec l'implant de resurfaçage du radius distal.

De préférence, la tige ulnaire forme un épaulement entre la base dudit pion et l'extrémité proximale de ladite portion distale médullaire.

Cet épaulement forme ainsi une butée limitative du mouvement de coulissement de la tige ulnaire par rapport à ladite tête d'articulation.

L'invention sera bien comprise, et d'autres caractéristiques et avantages de celle-ci apparaîtront, en référence au dessin schématique annexé, représentant, à titre d'exemples non limitatifs, une forme de réalisation préférée de l'implant de resurfaçage concerné et une forme de réalisation préférée d'une prothèse à articulation radio-ulnaire concernée.
La figure 1 est une vue en perspective de l'implant de resurfaçage ;
la figure 2 en est une vue d'extrémité, par son côté distal ;
la figure 3 en est une vue de côté, par son côté dorsal, avec repérage d'un axe de symétrie S d'une surface articulaire qu'il comprend et repérage d'un axe central C d'une tige médullaire d'ancrage, qu'il comprend également ;
la figure 4 en est une vue de côté, par son côté interne, également avec repérage desdits axe de symétrie S et axe central C ; et
la figure 5 en est une vue après implantation sur l'extrémité distale d'un radius, cette figure 5 montrant également les os d'une main et d'un poignet ;
la figure 6 est une vue en perspective d'une prothèse à articulation radio-ulnaire prothétique incluant ledit implant de resurfaçage, les différents éléments qu'inclut cette prothèse étant représentés de manière dégagée les unes des autres ;
la figure 7 est une vue de cette prothèse en perspective, après engagement desdits éléments ;
la figure 8 en est une vue en perspective, avec coupe selon un plan médian interne-externe ; et
la figure 9 en est une vue de côté, illustrant en traits fins les possibilités de débattement en pivotement et en translation d'une tige ulnaire qu'inclut cette prothèse.

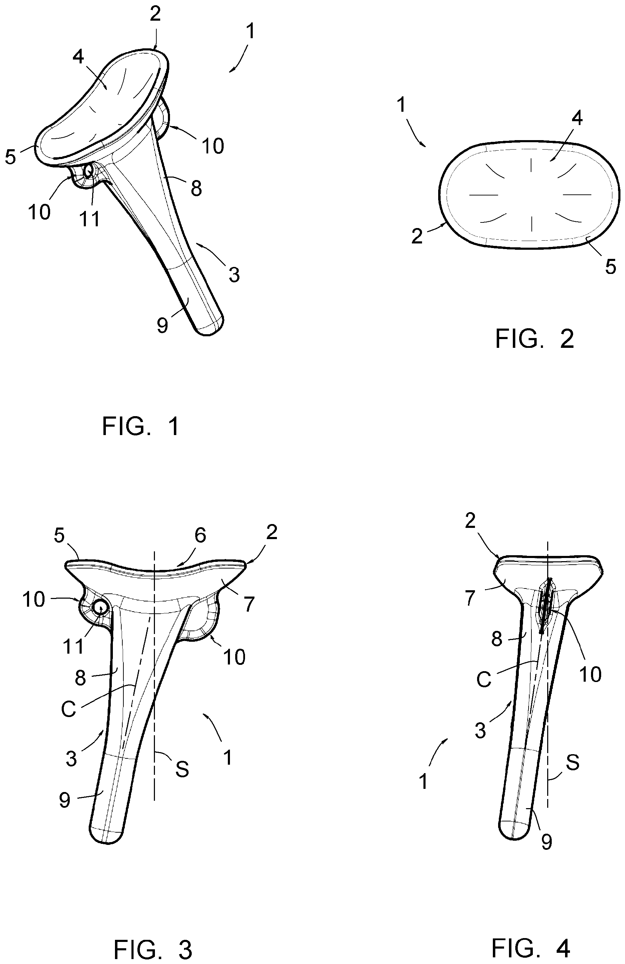

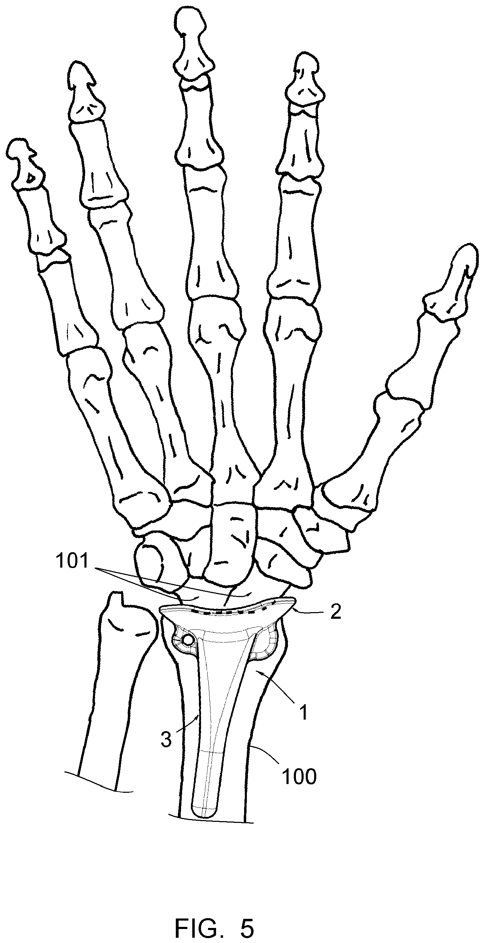

Les figures 1 à 4 représentent un implant 1 de resurfaçage du radius distal, permettant, ainsi que le montre la figure 5, de réaliser la reconstruction d'un radius distal 100, ou de remplacer l'articulation radio-carpienne native en cas de fracture complexe, en particulier chez un patient âgé pour qui l'ostéosynthèse est exclue.

Cet implant 1 est monobloc et à un seul composant, et comprend un plateau 2 et une tige 3 d'ancrage au radius 100, solidaire de ce plateau 2.

Le plateau 2 présente vu de face (cf. figure 2), une forme ovale occupée essentiellement par une surface d'articulation concave 4, destinée à venir directement en relation d'articulation avec les os 101 de la première rangée d'os du carpe, comme visible sur la figure 5. Il présente un rebord périphérique 5 arrondi, non enveloppant pour la surface d'articulation 3, et comprend deux échancrures arrondies 6 au niveau de ses bords longitudinaux, cf. figure 3. En outre, il présente, tant dans le sens de sa longueur (cf. figure 3) que dans le sens de sa largeur (cf. figure 4), une face 7 de forme arrondie et convexe sur son côté relié à la tige d'ancrage 3.

La longueur du plateau est comprise entre 25 mm et 35 mm et sa largeur est comprise entre 13 mm et 23 mm.

La tige d'ancrage 3 est effilée et destinée à être insérée dans le canal médullaire du radius 100. Elle a une longueur comprise entre 30 mm et 50 mm, et présente une portion métaphysaire 8 de forme conique aplatie et une portion diaphysaire de forme générale cylindrique 9. La portion métaphysaire 8 présente des faces planes dont une est tournée vers le côté dorsal de l'implant 1 (sur la droite de la figure 4) et l'autre est tournée vers le côté palmaire de cet implant (sur la gauche de cette figure 4).

De plus, la tige 3 présente une double inclinaison par rapport à l'axe de symétrie S du plateau 2, à savoir une inclinaison vers le côté interne de l'implant 1 (vers la gauche sur la figure 3), telle que l'axe central C de cette tige 3 forme un angle de 11° avec cet axe de symétrie S, et une inclinaison vers le côté palmaire de l'implant (vers la gauche sur la figure 4), telle que l'axe central C de cette tige 3 forme un angle de 8° avec cet axe de symétrie S.

La tige 3 comporte un revêtement de surface favorisant l'ostéo-intégration, en particulier un revêtement bicouche de titane poreux et d'Hydroxyapatite.

L'implant 1 comprend en outre deux ailettes 10 au niveau de la zone de raccordement du plateau 2 et de la tige 3, dont une est située sur le côté interne de l'implant 1 et l'autre sur le côté externe de cet implant. Chaque ailette présente une forme à deux bords plus ou moins perpendiculaires, raccordés l'un à l'autre par un bord intermédiaire arrondi. Les bords périphériques des ailettes 10 sont acérés, comme visible sur la figure 4, rendant possible l'insertion de l'implant 1 dans le radius 100 par impaction.

L'ailette 10 située sur le côté interne de l'implant 1 est percée d'un trou 11 de passage d'un ou plusieurs fils de suture.

En pratique, après résection adéquate de l'extrémité distale du radius 100, la tige 3 est introduite dans le canal médullaire de l'os et l'implant 1 est impacté jusqu'à venue de la face 7 du plateau au contact ou à proximité de l'os. Lors de cette impaction, les bords acérés des ailettes 10 s'insèrent dans l'os spongieux latéral de l'os et assurent un bon calage de l'implant 1 en rotation par rapport à l'os ; la forme précitée de tige 3 assure un bon remplissage de la portion métaphysaire du radius 100 ; l'implant conserve un caractère peu invasif compte tenu de la longueur relativement limitée de sa tige 3 ; la forme arrondie et convexe de la face 7 du plateau permet à l'implant 1 de venir s'insérer au mieux sur l'extrémité distale du radius 100.

Le trou 11 facilite le cas échéant la remise en place d'un fragment interne du radius, grâce au fil ou aux fils de suture qu'il permet de positionner.

Globalement, la combinaison de la forme ovale du plateau 2 et de la double inclinaison de la tige 3 confère à l'implant 1 une parfaite adaptation à l'anatomie de l'articulation radio-carpienne et permet de parfaitement restaurer cette articulation, sans générer d'inflammation ni de gêne ni de douleurs au patient. En particulier, cette forme spécifique favorise la stabilité de la première rangée des os 101 du carpe par rapport à l'implant 1 et assure une mobilité optimale de ces os par rapport au plateau 2.

Cette configuration permet en outre une résection a minima du radius 100 et de préserver l'échancrure sigmoïde de ce radius distal et l'articulation du radius distal à la tête de l'ulna distal, ou d'associer à la mise en place de l'implant une résection arthroplastique de la tête ulnaire, dite intervention de "Darrach".

Une fois l'implant en position dans l'os, un ancrage secondaire sera obtenu par croissance des cellules osseuses sur et dans le revêtement de surface que comporte la tige 3.

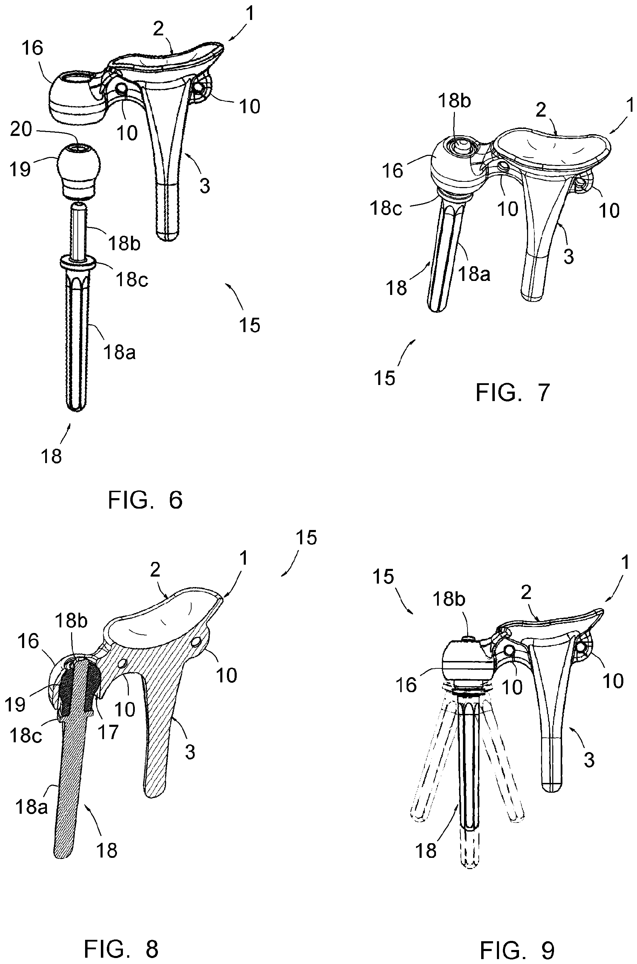

L'implant 1 selon les figures 1 à 5 est dépourvu d'articulation radio-ulnaire ; en référence aux figures 6 à 9, il apparaît que cet implant 1 peut faire partie d'une prothèse 15 à articulation radio-ulnaire prothétique.

Par simplification, les éléments ou parties déjà décrits en référence aux figures 1 à 5, qui se retrouvent sur les figures 6 à 9, ne seront pas à nouveau décrits et seront désignés par les mêmes références numériques.

Dans la prothèse 15, l'implant 1 est solidaire d'une portion annulaire 16 définissant intérieurement un siège 17 en forme de portion de sphère, et cette prothèse 15 comprend une tige ulnaire 18 et une tête 19 d'articulation de la tige ulnaire 18 par rapport à l'implant 1.

Dans l'exemple représenté, la portion annulaire 16 prolonge l'une des deux ailettes d'ancrage 10 de l'implant 1 et est solidaire de cette ailette 10, formant ainsi corps avec l'implant 1.

La portion annulaire 16 prend une forme externe sphérique sur son côté proximal, et est donc refermée sur ce côté, conférant au siège 17 une surface étendue sur ce côté proximal. Sur son côté distal, elle délimite une ouverture d'un diamètre légèrement supérieur à celui e la tête 19

La tige ulnaire 18 inclut une portion distale médullaire 18a, destinée à être insérée dans le canal médullaire de l'ulna, un pion proximal 18b de forme générale cylindrique, et une collerette 18c entre cette portion distale médullaire 18a et ce pion proximal 18b.

La tête 19 présente une face extérieure sphérique, destinée à être reçue contre le siège 17 avec glissement, et un alésage axial 20, permettant l'engagement de cette tête sur le pion proximal 18b, de manière ajustée mais avec possibilité de coulissement.

Comme le montre la figure 9, le glissement de la tête 19 contre le siège 17 permet un débattement de la tige ulnaire 18 par rapport à l'implant 1, de préférence sur environ 40 degrés au total, et, simultanément, le coulissement de cette tête le long du pion 18b permet un coulissement de cette tige ulnaire 18 par rapport à l'implant 1 selon l'axe de ce pion 18b.

L'épaulement que forme la collerette 18c entre la base du pion 18b et l'extrémité proximale de la portion distale médullaire 18a constitue une butée limitative du mouvement de coulissement de la tige ulnaire 18 par rapport à la tête d'articulation 19.

L'invention fournit ainsi un implant de resurfaçage du radius distal et une prothèse à articulation radio-ulnaire, incluant cet implant, qui présentent des avantages déterminants par rapport aux implants et prothèses homologues de la technique antérieure.

L'invention a été décrite ci-dessus en référence à des formes de réalisation fournies à titre de purs exemples. Il va de soi qu'elle n'est pas limitée à ces formes de réalisation mais qu'elle s'étend à toutes les autres formes de réalisation couvertes par les revendications ci-annexées.

## Revendications

1. Implant (1) de resurfaçage du radius (100) distal, monobloc et à un seul composant, comprenant un plateau (2) formant une surface d'articulation concave et une tige d'ancrage (3) effilée, solidaire de ce plateau (2), destinée à être insérée dans le canal médullaire de l'extrémité distale du radius (100) ;
- le plateau (2) présente vu de face, une forme ovale occupée essentiellement par la surface d'articulation concave (4), et est dépourvu de rebord périphérique enveloppant ;
- la tige d'ancrage (3) présente une double inclinaison par rapport au plateau (2), à savoir une inclinaison vers le côté interne de l'implant (1) et une inclinaison vers le côté palmaire de l'implant (1) ;
**caractérisé en ce que** :
- ladite surface d'articulation concave est destinée à venir directement en relation d'articulation avec les os (101) de la première rangée d'os du carpe,
- le plateau (2) est symétrique par rapport à un axe de symétrie (S) ;
- l'inclinaison de la tige d'ancrage (3) vers le côté interne est telle que l'axe central (C) de cette tige forme un angle allant de 6 à 16° avec cet axe de symétrie (S), et l'inclinaison de la tige d'ancrage (3) vers le côté palmaire de l'implant (1) est telle que l'axe central (C) de cette tige forme un angle allant de 4 à 12° avec cet axe de symétrie (S).

2. Implant de resurfaçage (1) selon la revendication 1, **caractérisé en ce que** l'inclinaison de la tige (3) vers le côté interne de l'implant (1) est telle que l'axe central (C) de cette tige forme un angle de 11° avec cet axe de symétrie (S), et **en ce que** l'inclinaison de la tige (3) vers le côté palmaire de l'implant (1) est telle que l'axe central (C) de cette tige forme un angle de 8° avec cet axe de symétrie (S).

3. Implant de resurfaçage (1) selon la revendication 1 ou la revendication 2, **caractérisé en ce que** le plateau (2) présente, tant dans le sens de sa longueur que dans le sens de sa largeur, une face (7) de forme arrondie et convexe sur son côté relié à la tige d'ancrage (3).

4. Implant de resurfaçage (1) selon l'une des revendications 1 à 3, **caractérisé en ce que** la tige d'ancrage (3) présente une portion métaphysaire (8) de forme conique aplatie et une portion diaphysaire (9) de forme générale cylindrique.

5. Implant de resurfaçage (1) selon l'une des revendications 1 à 4, **caractérisé en ce que** la portion métaphysaire (8) de la tige (3) présente des faces planes dont une est tournée vers le côté dorsal de l'implant (1) et l'autre est tournée vers le côté palmaire de cet implant.

6. Implant de resurfaçage (1) selon l'une des revendications 1 à 5, **caractérisé en ce que** la tige (3) a une longueur comprise entre 30 mm et 50 mm.

7. Implant de resurfaçage (1) selon l'une des revendications 1 à 6, **caractérisé en ce que** la tige d'ancrage (3) comporte un revêtement de surface favorisant l'ostéo-intégration, en particulier un revêtement bicouche de titane poreux et d'Hydroxyapatite.

8. Implant de resurfaçage (1) selon l'une des revendications 1 à 7, **caractérisé en ce qu'**il comprend deux ailettes d'ancrage (10) au niveau de la zone de raccordement du plateau (2) et de la tige d'ancrage (3), une ailette (10) étant située sur le côté interne de l'implant (1) et l'autre ailette (10) étant située sur le côté externe de cet implant.

9. Implant de resurfaçage (1) selon la revendication 8, **caractérisé en ce que** chaque ailette (10) présente une forme à deux bords plus ou moins perpendiculaires, raccordés l'un à l'autre par un bord intermédiaire arrondi.

10. Implant de resurfaçage (1) selon la revendication 8 ou la revendication 9, **caractérisé en ce qu'**au moins une de ces ailettes (10) présente un bord périphérique acéré, rendant possible l'insertion de l'implant (1) dans un radius (100) par impaction.

11. Implant de resurfaçage (1) selon l'une des revendications 8 à 10, **caractérisé en ce qu'**au moins une ailette (10), en particulier celle située sur le côté interne de l'implant (1), est percée d'un trou (11) de passage d'un ou plusieurs fils de suture.

12. Prothèse (15) à articulation radio-ulnaire prothétique, **caractérisée en ce qu'**elle comprend l'implant (1) de resurfaçage du radius distal selon l'une des revendications 1 à 11, cet implant (1) étant solidaire d'une portion annulaire (16) définissant intérieurement un siège (17) en forme de portion de sphère, et **en ce que** la prothèse (15) comprend :
- une tige ulnaire (18) incluant une portion distale médullaire (18a), destinée à être insérée dans le canal médullaire de l'ulna, et un pion proximal (18b) de forme générale cylindrique ; et
- une tête (19) d'articulation de la tige ulnaire (18) par rapport à l'implant (1) de resurfaçage du radius distal, présentant une face extérieure sphérique ou en portion de sphère destinée à être reçue contre ledit siège (17) avec glissement, et un alésage axial (20), permettant l'engagement de cette tête sur ledit pion (18b), de manière ajustée mais avec possibilité de coulissement.

13. Prothèse (15) selon la revendication 12, **caractérisée en ce que** le débattement de la tige ulnaire (18) par rapport à l'implant (1) de resurfaçage du radius distal est de l'ordre de 20 degrés par rapport à une position neutre d'alignement de l'axe de la tige ulnaire (18) avec l'axe de révolution dudit siège (17), soit un débattement d'environ 40 degrés d'une position d'inclinaison maximale de la tige ulnaire (18) à une position d'inclinaison maximale opposée.

14. Prothèse (15) selon la revendication 12 ou la revendication 13, incluant l'implant (1) selon l'une des revendications 9 à 12, **caractérisée en ce que** ladite portion annulaire (16) prolonge l'une des deux ailettes d'ancrage (10) que comprend l'implant (1) de resurfaçage du radius distal et forme corps avec cet implant.

15. Prothèse (15) selon l'une des revendications 12 à 14, **caractérisée en ce que** la tige ulnaire (18) forme un épaulement (18c) entre la base dudit pion (18b) et l'extrémité proximale de ladite portion distale médullaire (18a).

## Patentansprüche

1. Implantat zum Flächewiederaufbau (1) des distalen Radius (100) mit einteiliger und einkomponentiger Struktur, mit einer Platte (2), die eine konkave Gelenkfläche Platte (2) bildet und einer mit dieser Platte (2) kraftschlüssig verbundenen verjüngten Verankerungsstange (3) in den Markraum des distalen Endes des Radius (100) eingeführt werden ;
- die Platte (2), von vorne gesehen, hat eine ovale Form, die im wesentlichen durch die konkave Gelenkfläche (4) besetzt ist, und wird von einem umhüllenden Umfangsrand beraubt;
- die Ankerstange (3) eine doppelte Neigung gegenüber der Platte (2) aufweist, nämlich eine Neigung zur Innenseite des Implantats (1) und eine Neigung zur Palmarseite des Implantats (1);
**Dadurch gekennzeichnet, daß**:
- die konkave Gelenkfläche soll direkt in eine Gelenkbeziehung mit den Knochen (101) der ersten Knochenreihe des Karpus kommen,
- die Platte (2) ist symmetrisch bezüglich einer Symmetrieachse (S);
- die Neigung der Ankerstange (3) zur Innenseite ist so, daß die Mittelachse (C) dieser Stange mit dieser Symmetrieachse (S) einen Winkel von 6 bis 16 ° und die Neigung des Ankerstange (3) zur Palmarseite des Implantats (1) ist so, dass die Mittelachse (C) dieser Stange mit dieser Symmetrieachse (S) einen Winkel von 4 bis 12 ° bildet.

2. Implantat zum Flächewiederaufbau (1) nach Anspruch 1, **dadurch gekennzeichnet, daß** die Neigung der Stange (3) zur Innenseite des Implantats derart ist, daß die Mittelachse (C) der Stange mit dieser Symmetrieachse einen Winkel von 11 ° bildet (S), und daß die Neigung der Stange (3) zur Palmarseite des Implantats (1) derart ist, daß die Mittelachse (C) dieser Stange mit dieser Symmetrieachse einen Winkel von 8 ° bildet (S).

3. Implantat zum Flächewiederaufbau (1) nach einem der Ansprüche 1 oder 2, **dadurch gekennzeichnet, daß** die Platte (2) in Richtung ihrer Länge sowie in Richtung ihrer Breite eine Abgerundete und konvexformige Fläche (7) an ihrer mit der Ankerstange (3) verbundenen Seite aufweist.

4. Implantat zum Flächewiederaufbau (1) nach einem der Ansprüche 1 bis 3, **dadurch gekennzeichnet, dass** die Verankerungsstange (3) einen metaphysären Abschnitt (8) mit abgeflachter konischer Form und einen diaphysären Abschnitt (9) von allgemein zylindrischer Form aufweist.

5. Implantat zum Flächewiederaufbau (1) nach einem der Ansprüche 1 bis 4, **dadurch gekennzeichnet, dass** der metaphysäre Abschnitt (8) der Stange (3) ebene Flächen aufweist, von denen eine der dorsalen Seite des Implantats (1) zugewandt ist, und die andere von der palmare Seite dieses Implantats zugewandt ist.

6. Implantat zum Flächewiederaufbau (1) nach einem der Ansprüche 1 bis 5, **dadurch gekennzeichnet, daß** die Stange (3) eine Länge zwischen 30 mm und 50 mm aufweist.

7. Implantat zum Flächewiederaufbau (1)nach einem der Ansprüche 1 bis 6, **dadurch gekennzeichnet, dass** die Verankerungsstange (3) eine Osteointegration fördernde Oberflächenbeschichtung umfasst, insbesondere eine zweischichtige Beschichtung aus porösem Titan und Hydroxyapatit.

8. Implantat zum Flächewiederaufbau (1) nach einem der Ansprüche 1 bis 7, **dadurch gekennzeichnet, dass** es am Verbindungsbereich der Platte (2) und der Verankerungsstange (3) zwei Verankerungsrippen (10) aufweist wobei die Rippe (10) auf der Innenseite des Implantats (1) und die andere Rippe (10) auf der Außenseite dieses Implantats angeordnet ist.

9. Implantat zum Flächewiederaufbau (1) nach Anspruch 8, **dadurch gekennzeichnet, daß** jede Rippe (10) eine Form mit zwei mehr oder weniger senkrechten Kanten aufweist, die durch eine abgerundete Zwischenkante miteinander verbunden sind.

10. Implantat zum Flächewiederaufbau (1) nach einem der Ansprüche 8 oder 9, **dadurch gekennzeichnet, dass** mindestens eine dieser Rippen (10) eine geschärfte Umfangskante aufweist, die es ermöglicht, das Implantat in einen Radius (100) pro Impaktion einzusetzen.

11. Implantat zum Flächewiederaufbau (1) nach einem der Ansprüche 8 bis 10, **dadurch gekennzeichnet, dass** mindestens eine an der Innenseite des Implantats (1) befindliche Rippe (10) ein Loch (11) zum Übergeben eines oder mehrerer Nähgarne.

12. Prothese (15) mit einem Radio-Ulnar-Prothesengelenk, **dadurch gekennzeichnet, dass** es das Implantat zum Flächewiederaufbau (1) des distalen Radius nach einem der Ansprüche 1 bis 11 umfasst, wobei dieses Implantat (1) kraftschlüssig verbunden mit einem ringförmigen Teil ausgebildet ist (16), die innen einen Sitz (17) in Form eines Teils einer Kugel definieren, und daß die Prothese (15) umfaßt:
- eine Ulnarstange (18) mit einem distalen Markabschnitt (18a), der dazu bestimmt ist, in den Markraum der Ulna eingeführt zu werden, und einen proximalen Zapfen (18b) von allgemein zylindrischer Form; und
- einen Kopf (19) zum Anlenken der Ulnarstange (18) in Bezug auf das Implantat zum Flächewiederaufbau (1) des distalen Radius mit einer äußeren kugelförmigen, oder teilweise kugelförmigen Fläche, die gleitend gegen den Sitz (17) aufgenommen werden soll, und eine axiale Bohrung (20), die es dem Kopf ermöglicht, auf dem Zapfen (18b) in einer angepassten, aber verschiebbaren Weise in Eingriff gebracht zu werden.

13. Prothese (15) nach Anspruch 12, **dadurch gekennzeichnet, dass** der Bewegungsbereich der Ulnarstange (18) gegenüber dem Implantat zum Flächewiederaufbau (1) des distalen Radius in der Größenordnung von 20 Grad gegenüber einer Neutralstellung der Ausrichtung der Achse der Ulnarstange (18) mit der Drehachse des Sitzes (17), dh ein Bewegungsbereich von etwa 40 Grad von einer Position der maximalen Neigung der Ulnarstange (18) zu einem entgegengesetzten Maximal Neigungsposition

14. Prothese (15) nach Anspruch 12 oder 13, umfassend das Implantat (1) nach einem der Ansprüche 9 bis 12, **dadurch gekennzeichnet, daß** der ringförmige Abschnitt (16) eine der beiden Rippen (10) verlängert, das Implantat zum Flächewiederaufbau (1) des distalen Radius umfasst und mit diesem Implantat einstückig ist.

15. Prothese nach einem der Ansprüche 12 bis 14, **dadurch gekennzeichnet, dass** der Ulnarschaft (18) eine Schulter (18c) zwischen dem Boden des Zapfens (18b) und dem proximalen Ende des medullären distalen Teils (18a) bildet.

## Claims

1. An implant (1) for resurfacing the distal radius (100), of integral and single component structure, comprising a plate (2) forming a concave articulation surface and a tapered anchoring rod (3) integral with this plate (2) intended to be inserted in the medullary canal of the distal end of the radius (100);
- the plate (2), as seen from the front, has an oval shape occupied essentially by the concave articulation surface (4), and is deprived of any enveloping peripheral rim;
- the anchor rod (3) has a double inclination with respect to the plate (2), namely an inclination towards the internal side of the implant (1) and an inclination towards the palmar side of the implant (1);
**characterized in that**:
- said concave articulation surface is intended to come directly into an articulation relation with the bones (101) of the first row of bones of the carpus,
- the plate (2) is symmetrical with respect to an axis of symmetry (S);
- the inclination of the anchor rod (3) towards the internal side is such that the central axis (C) of this rod forms an angle ranging from 6 to 16° with this axis of symmetry (S), and the inclination of the anchor rod (3) towards the palmar side of the implant (1) is such that the central axis (C) of this stem forms an angle ranging from 4 to 12° with this axis of symmetry (S).

2. The implant (1) for resurfacing as claimed in claim 1, **characterized in that** the inclination of the rod towards the internal side of the implant is such that the central axis of the rod forms an angle of 11° with this axis of symmetry (S), and **in that** the inclination of the rod (3) towards the palmar side of the implant (1) is such that the central axis (C) of this rod forms an angle of 8° with this axis of symmetry (S).

3. The implant (1) for resurfacing as claimed in claim 1 or claim 2, **characterized in that** the plate (2) has, in the direction of its length as well as in the direction of its width, a face (7) a rounded and convex shape on its side connected to the anchor rod (3).

4. The implant (1) for resurfacing according to one of claims 1-3, **characterized in that** the anchoring rod has a metaphyseal portion (8) of flattened conical shape and a diaphyseal portion (9) of generally cylindrical shape.

5. The implant (1) for resurfacing according to one of claims 1-4, **characterized in that** the metaphyseal portion (8) of the rod (3) has planar faces, one of which facing the dorsal side of the implant (1) and the other of which facing the palmar side of this implant.

6. The implant (1) for resurfacing according to one of claims 1-5, **characterized in that** the rod (3) has a length of between 30 mm and 50 mm.

7. The implant (1) for resurfacing according to one of claims 1-6, **characterized in that** the anchoring rod (3) comprises a surface coating promoting osteointegration, in particular a two-layer coating of porous titanium and Hydroxyapatite.

8. The implant (1) for resurfacing according to one of claims 1-7, **characterized in that** it comprises two anchoring fins (10) at the area of connection of the plate (2) and of the anchoring rod (3), one fin (10) being located on the inner side of the implant (1) and the other fin (10) being located on the outer side of this implant.

9. The implant (1) for resurfacing according to claim 8, **characterized in that** each fin (10) has a shape with two edges more or less perpendicular, connected to each other by a rounded intermediate edge.

10. The implant (1) for resurfacing as claimed in claim 8 or claim 9, **characterized in that** at least one of these fins (10) has a sharpened peripheral edge making it possible to insert the implant in a radius (100) per impaction.

11. The implant (1) for resurfacing as claimed in one of claims 8-10, **characterized in that** at least one fin (10), in particular the one located on the internal side of the implant (1), comprises a hole (11) for passing one or more sutures.

12. A prosthesis (15) with a radio-ulnar prosthetic joint, **characterized in that** it comprises the implant (1) for resurfacing the distal radius according to one of claims 1 to 11, this implant (1) being integral with an annular portion (16) internally defining a seat (17) in the form of a portion of a sphere, and **in that** the prosthesis (15) comprises:
- an ulnar stem (18) including a distal medullary portion (18a), intended to be inserted in the medullary canal of the ulna, and a proximal peg (18b) of generally cylindrical shape; and
- a head (19) for articulating the ulnar stem (18) with respect to the implant (1) for resurfacing (1) the distal radius, having an external spherical partly spherical surface intended to be slidingly received against said seat (17), and an axial bore (20) enabling said head to be engaged on said peg (18b) in an adjusted but slidable manner.

13. The prosthesis (15) as claimed in claim 12, **characterized in that** the range of movement of the ulnar stem with respect to the implant (1) for resurfacing the distal radius is of the order of 20 degrees with respect to a neutral position of alignment of the axis of the ulnar stem (18) with the axis of revolution of said seat (17), i.e. a range of movement of approximately 40 degrees from a position of maximum inclination of the ulnar stem (18) to an opposite maximum inclination position.

14. The prosthesis (15) according to claim 12 or claim 13, including the implant (1) according to one of claims 9 to 12, **characterized in that** said annular portion (16) prolongates one of the two fins (10) which comprises the implant (1) for resurfacing (1) the distal radius and is integral with this implant.

15. The prosthesis as claimed in one of claims 12-14, **characterized in that** the ulnar stem (18) forms a shoulder (18c) between the base of said peg (18b) and the proximal end of said medullar distal portion (18a).
